(19) Europäisches Patentamt
European Patent Office
Office européen des brevets

(11) Veröffentlichungsnummer: **0 012 725**
**B1**

(12) **EUROPÄISCHE PATENTSCHRIFT**

(45) Veröffentlichungstag der Patentschrift: **29.02.84**

(51) Int. Cl.³: **C 07 D 241/52,**
A 61 K 31/495, A 23 K 1/16

(21) Anmeldenummer: **79810181.2**

(22) Anmeldetag: **13.12.79**

(54) **Chinoxalin-Di-N-oxid-Derivate, Verfahren zu ihrer Herstellung, Mittel diese enthaltend und ihre Verwendung.**

(30) Priorität: **19.12.78 CH 12887/78**
**05.07.79 CH 6295/79**
**20.11.79 CH 10341/79**

(43) Veröffentlichungstag der Anmeldung:
**25.06.80 Patentblatt 80/13**

(45) Bekanntmachung des Hinweises auf die Patenterteilung:
**29.02.84 Patentblatt 84/9**

(84) Benannte Vertragsstaaten:
**BE CH DE FR GB NL SE**

(56) Entgegenhaltungen:
**DE - A - 2 701 707**
**FR - A - 2 085 717**

(73) Patentinhaber: **CIBA-GEIGY AG**
**Patentabteilung Postfach**
**CH-4002 Basel (CH)**

(72) Erfinder: **Schmid, Wolfgang**
**Birsmattweg 32**
**CH-4106 Therwil (CH)**

**Die Akte enthält technische Angaben, die nach dem Eingang der Anmeldung eingereicht wurden und die nicht in dieser Patentschrift enthalten sind.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 012 725 B1

# Chinoxalin-di-N-oxid-Derivate, Verfahren zu ihrer Herstellung, Mittel diese enthaltend und ihre Verwendung

Die vorliegende Erfindung betrifft neue Chinoxalin-di-N-oxid-Derivate, Verfahren zu ihrer Herstellung, die neuen Verbindungen enthaltende Mittel und ihre Verwendung zur Bekämpfung pathogener Mikroorganismen sowie als Futterzusatz für Haus- und Nutztiere.

Die neuen Chinoxalin-di-N-oxid-Derivate entsprechen der allgemeinen Formel I

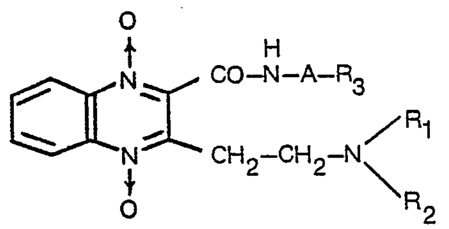

(I)

in welcher

$R_1$ und $R_2$ unabhängig voneinander geradkettiges oder verzweigtes Alkyl mit 1 bis 4 Kohlenstoffatomen oder zusammen mit dem N-Atom einen gegebenenfalls durch Alkyl mit 1 bis 4 Kohlenstoffatomen substituierten heterocyclischen Ring mit 4 bis 5 Ring-Kohlenstoffatomen und gegebenenfalls einem Sauerstoffatom als weiterem Heteroatom,

$R_3$ Wasserstoff, Methoxy, Methylthio, Hydroxy, Fluor, Chlor, Brom oder Cyan,

A Alkylen mit 1 bis 4 Kohlenstoffatomen bedeuten, mit der Massgabe, dass, wenn $R_3$ verschieden von Cyan ist, A 1,2-Aethylen bedeutet, einschliesslich ihrer Säureadditionssalze (=Verbin=dungen der Formel Ia; siche S.5).

Unter Alkyl sind Methyl, Aethyl und die Isomeren der Propyl- und der Butylgruppe zu verstehen.

Als Beispiele für die Säureanionen der Säureadditionssalze sind Azetat, Citrat, Maleat, Lactat, Fumarat, Pamoat, Tosylat, Bromid und vorzugsweise Chlorid sowie Salze mit Ionenaustauschern, wie z.B. Amberlit 15 zu nennen.

Stoffe, die den erfindungsgemässen Verbindungen der Formel I strukturell ähnlich sind, sind bereits aus den DE—A—1 670 935 und DE—A—1 620 114 sowie der GB—A—1 223 720 bekannt. Die erfindungsgemässen Verbindungen der Formel I bzw. Ia sind den bekannten Verbindungen bei der Bekämpfung pathogener Mikroorganismen weit überlegen und zeichnen sich ihnen gegenüber besonders durch stärkere therapeutische Wirksamkeit sowie geringe Toxizität aus. Ferner sind sie in vorteilhafter Weise sehr leicht sowohl in Wasser als auch in organischen Lösungsmitteln löslich.

Die erfindungsgemässen Verbindungen der Formel I besitzen eine gute mikrobizide Wirkung und eignen sich für die Bekämpfung pathogener Mikroorganismen im veterinärmedizinischen Bereich. Sie zeichnen sich durch eine gute Wirkung gegen durch E. coli hervorgerufene Luftwegerkrankungen beim Geflügel aus. Ausserdem sind sie brauchbar zur Bekämpfung von Infektionen des Intestinaltraktes, z.B. der Schweinediarrhoe, sowie von Infektionen des Urogenitalsystems. Darüberhinaus besitzen sie gute wachstumsfördernde Eigenschaften bei Haus- und Nutztieren wie Schweinen, Geflügel und Wiederkäuern.

In der DE—A—2 701 707, der DE—A—2 035 480 und der FR—A—2 085 717 werden 3 - Methyl - 2 - chinoxalincarboxamid - di - N - oxid - Derivate als antibakterielle Präparate und Wachstumsförderer zur Tierzucht vorgeschlagen. Demgegenüber weisen die 3 - Aminoäthyl - 2 - chinoxalincarboxamid - di - N - oxide der Formel I vorliegender Erfindung signifikante Vorteile in der Wachstumsförderung bei gleichzeitig deutlich gesenkter Warmblüter-Toxizität auf.

In diesen letzten Eigenschaften zeichnen sich besonders die Verbindungen der Formel I b durch gute Wirksamkeit aus,

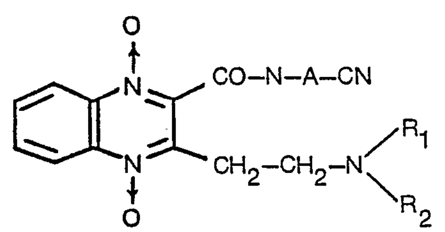

(Ib)

in welcher $R_1$ und $R_2$ unabhängig voneinander $C_1$—$C_4$-Alkyl oder zusammen mit dem N-Atom einen gegebenenfalls durch $C_1$—$C_4$-Alkyl substituierten heterocyclischen Ring mit 4 bis 5 Ring-Kohlenstoffatomen und gegebenenfalls einem Sauerstoffatom als weiterem Heteroatom darstellen, und ihre Säureadditionssalze.

Unter den Verbindungen der Formel Ib sind mit besonders günstigemn Toxizitätswerten bei Schwein und Huhn folgende Verbindungen zu nennen:

Verbindungstabelle 1:

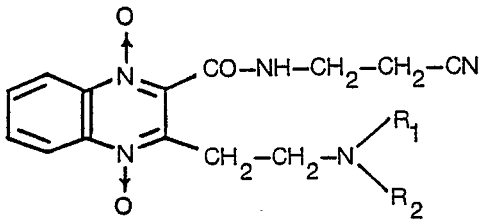

(V)

oder

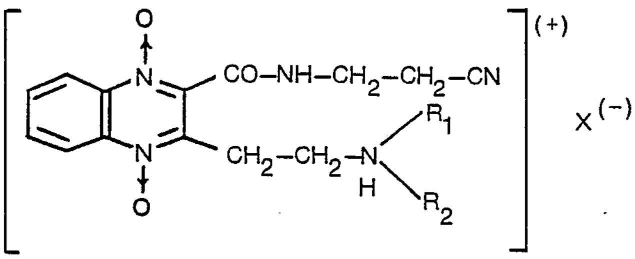

(VI)

| Verb.-Nr. | 1 | 2 | 3 | 4 | 5 |
|---|---|---|---|---|---|
| $R_1$: | 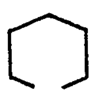 | $CH_3$ | 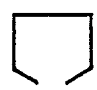 | 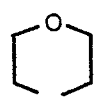 | $C_2H_5$ |
| $R_2$: | | $CH_3$ | | | $C_2H_5$ |
| X : | Cl | Cl | Cl | Cl | – |

Eine weitere Verbindung mit ebenfalls guter wachstumsfördernder Wirksamkeit und geringer Toxizität ist folgende:

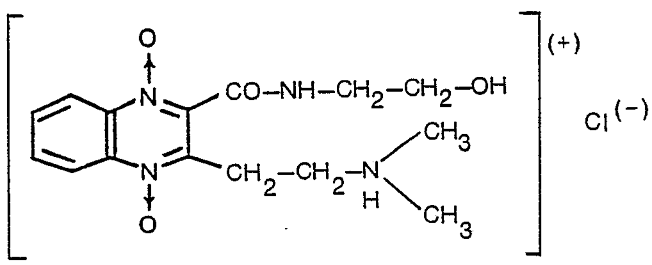

(VII)

Die erfindungsgemässen Wirkstoffe können entsprechend der beabsichtigten Verwendung als solche oder in Kombination mit inerten Trägerstoffen bzw. Verdünnungsmitteln in Form von Lösungen, Emulsionen, Suspensionen, Pulvern, Tabletten, Bolussen und Kapseln peroral, abomasal oder via Injektion den Tieren direkt und zwar als Einzeldosis wie auch wiederholt verabreicht werden. Die Wirkstoff bzw. sie enthaltende Gemische können auch dem Futter oder den Tränken zugesetzt werden oder in sogenannten Futtervormischungen enthalten sein.

Die gute therapeutische Wirksamkeit der erfindungsgemässen Verbindungen der Formel I bzw. Ia konnte sowhol in vitro als auch insbesondere am Tier bei akuten bakteriellen Infektionen nach oraler sowie subcutaner Applikation festgestellt werden. Das Wirkungsspektrum der Verbindungen umfasst grampositive und gramnegative Bakterien.

Die Verbindungen der Formel I sind nach bekannten Methoden aus 1,4-Dioxido-3-methyl-chinoxalin (2)-carbonsäureamiden [vgl. Organic Reactions, Vol I (1942) 312] folgendermassen herstellbar:

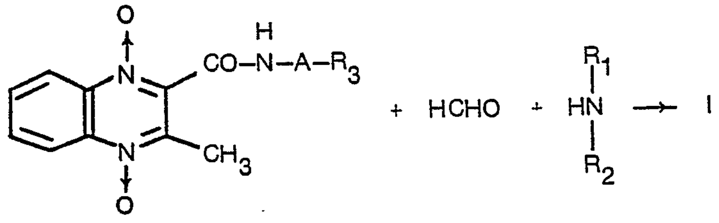

(II) (III)

bzw. ihre Säureadditionssalze:

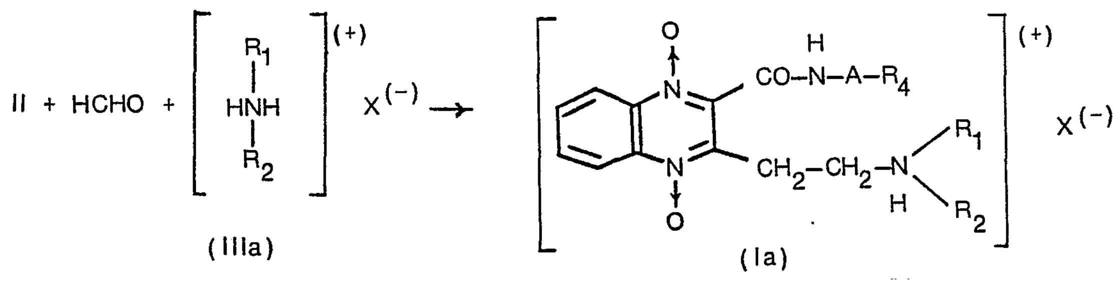

(IIIa) (Ia)

Die Salze der Formel Ia rand die freien Verbindungen der Formel I wie folgt überführbar:

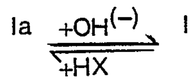


In den angeführten Formeln haben die Reste $R_1$ bis $R_3$ die eingangs unter Formel I angegebenen Bedeutungen und X stellt das Anion einer anorganischen oder organischen Säure dar.

Die Umsetzung wird in für die Reaktionspartner inerten organischen Lösungsmitteln wie beispielsweise Formamid, N-Methylformamid, Dimethylformamid, Dimethylsulfoxid, Tetrahydrofuran, Dioxan, Toluol, Propionitril, Butyronitril, Benzonitril, Methylcellosolve oder vorzugsweise Acetonitril durchgeführt.

Die Reaktion erfolgt bei Temperaturen von 20 bis 150°C, vorzugsweise 40 bis 80°C.

Die für die Herstellung der erfindungsgemässen Verbindungen der Formel I verwendeten Ausgangsstoffe der Formel II sind teilweise bereits beschrieben (vgl. DE—A—1 670 935, DE—A—211 710, GB—A—1 308 370, CH—A—7718/77). Stellt $R_3$ in Formel II Fluor dar, kann die entsprechende Verbindung nach einem in den vorgenannten Beschreibungen aufgeführten Verfahren hergestellt werden. Für den Fall, dass $R_3$ Brom, $R_1$ Wasserstoff und A Aethylen bedeuten (s. Formel II'), wird folgendes neues Verfahren zur Herstellung verwendet:

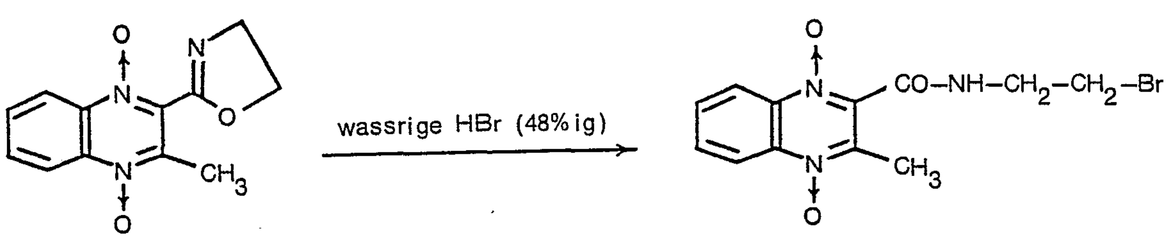


(IV) (II')

Bedeutet $R_3$ Chlor, lässt sich die entsprechende Verbindung der Formel II'' (Cl anstelle von Br in Formel II'), die bereits in der DE—A—2 111 710 genannt ist, ebenfalls nach dem oben beschriebenen Verfahren durch Umsetzung von 2 - [Oxazolinyl(2')] - 3 - methyl - chinoxalin - 1,4 - di - N - oxid (DE—A—2 111 710/Beispiel 21) mit konzentrierter, wässriger Salzsäure herstellen.

Ebenfalls bekannt sind die als Ausgangsstoffe der Formel III bzw. IIIa verwendeten sekundären Amine bzw. deren Säureadditionssalze, von denen als Beispiele zu nennen sind:

Dimethylamin, Diäthylamin, Di-n-propylamin, Di-isopropylamin, Di-n-butylamin, Pyrrolidin, Piperidin und Morpholin sowie deren Säureadditionssalze.

Der in dem Verfahren zur Herstellung der erfindungsgemässen Verbindungen der Formel I bzw. Ia verwendete Formaldehyd kann sowohl in Form einer wässrigen Lösung als auch in Form von Formaldehyd abspaltenden festen Mitteln wie beispielsweise Paraformaldehyd verwendet werden.

Beispiel 1

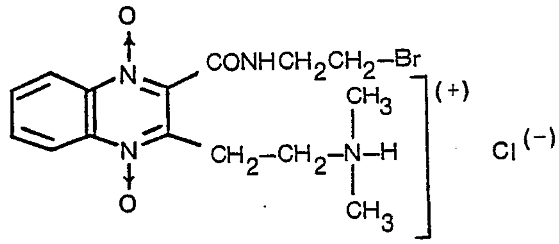

a) 49 g (0.2 Mol) 2 - [Oxazolinyl(2')] - 3 - methyl - chinoxalin - 1,4 - di - N - oxid gibt man in 200 ml 48 %ige, wässrige Bromwasserstoffsäure und rührt anschliessend 1 Stunde bei Raumtemperatur weiter. Versetzt man nun dieses Gemisch mit 500 g Eis, fällt ein farbloses, kristallines Produkt aus, das abfiltriert und bei Raumtemperatur über Phosphorpentoxid getrocknet wird. Man erhält so 63.1 g (96.7%) 1,4 - Dioxido - 3 - methyl - chinoxalin(2) - N - (2' - bromaethyl) - carbonsäureamid, das bei 165°C unter Zersetzung schmilzt.

b) 24.5 g (0.075 Mol) 1.4 - Dioxido - 3 - methyl - chinoxalin(2) - N - (2' - bromaethyl) - carbonsäureamid, 2.3 g Paraformaldehyd und 6 g Dimethylaminhydrochlorid gibt man mit 180 ml Acetonitril zusammen und erwärmt im Oelbad auf 100°C. Die Suspension ergibt allmählich eine klare Lösung, die man unter Rühren weitere 20 Stunden bei gleicher Temperatur hält. Kühlt man auf 10°C ab, so kristallisiert ein Produkt aus. Das Gemisch wird nochmals auf 60°C erwärmt und dann abfiltriert. Man erhält so 14.6 g (46.5%) 1.4 - Dioxido - 3 - (2' - dimethylaminoaethyl) - chinoxalin(2) - N - (2'' - bromaethyl) - carbonsäureamid - hydrochlorid, das bei 164—166°C unter Zersetzung schmilzt.

Beispiel 2

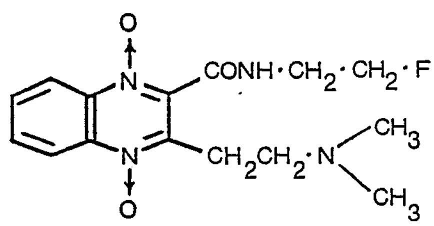

a) Zu einer Suspension von 4.4 g (0.03 Mol) N-2-Fluoräthylacetessigsäureamid und 4 g Benzfuroxan in 30 ml Methanol tropft man 2.2 g n-Butylamin während 10 Minuten zu, wobei die Temperatur auf 35°C ansteigt und für kurze Zeit eine klare, dunkelgefärbte Lösung entsteht. Man rührt bei gleicher Temperatur im Wasserbad weiter, kühlt nach 10 Stunden auf 20°C ab, saugt das ausgefallene kristalline Produkt ab und wäscht es mit wenig kaltem Methanol. Es lässt sich aus Methanol umkristallisieren. Die erhaltenen 6 g (75.9%) reines, kristallines 1.4 - Dioxido - 3 - methyl - chinoxalin - (2) - N - (2' - fluoräthyl) - carbonsäureamid schmelzen bei 200—202°C.

b) 52.4 g (0.2 Mol) 1.4 - Dioxido - 3 - methyl - chinoxalin(2) - N - (2' - fluoraethyl)carbonsäureamid, 6 g Paraformaldehyd und 16 g Dimethylaminhydrochlorid erwärmt man in 500 ml Acetonitril im Oelbad auf 100°C. Die Suspension ergibt allmählich eine klare Lösung, die man unter Rühren weitere 20 Stunden bei gleicher Temperatur hält, wobei nach 10 Stunden sich langsam ein kristallines Produkt abzuscheiden beginnt. Filtriert man dieses dann aus der warmen Mischung ab und kristallisiert es aus Aethanol um, so erhält man 45.6 g (63.7%) 1.4 - Dioxido - 3 - (2' - dimethylaminoaethyl) - chinoxalin(2) - N - (2'' - fluoräthyl) - carbonsäure - amid - hydrochlorid, das bei 182—184°C unter Zersetzung schmilzt.

c) 3.6 g (0.01 Mol) 1.4 - Dioxido - 3 - (2' - dimethylaminoäthyl) - chinoxalin(2) - N - (2'' - fluoroäthyl) - carbonsäureamid - hydrochlorid löst man in einem Gemisch von 30 ml Wasser und 30 ml Methylenchlorid auf, tropft unter Rühren 10 ml 1—N Natronlauge zu und trennt sofort die Methylenchloridphase ab. Aus der über Natriumsulfat getrockneten Lösung erhält man 2.7 g (84.4%) reines, kristallines 1.4 - Dioxido - 3 - (2' - dimethylaminoäthyl) - chinoxalin(2) - N - (2'' - fluoräthyl) - carbonsäureamid vom Schmelzpunkt 166—167°C.

Beispiel 3

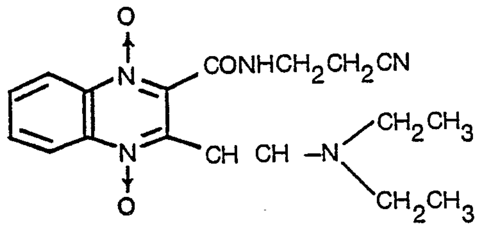

27,2 g (0,1 Mol) 1,4 - Dioxido - 3 - methyl - chinoxalin(2) - N - (2′ - cyanäthyl) - carbonsäureamid werden mit 20 g (0,2 Mol) 30%iger, wässriger Formaldehyd-Lösung und 14,6 g (0,2 Mol) Diäthylamin in 250 ml Acetonitril suspendiert. Anschliessend rührt man 16 Stunden bei 40°C und dampft dann die gebildete klare Lösung ein. Nach Zugabe von Methanol zum öligen Rückstand scheiden sich aus der dunkelbraun gefärbten, klaren Lösung 23,4 g (65,5%) vom 1,4 - Dioxido - 3 - (2′ - diäthylaminoäthyl) - chinoxalin(2) - N - (2′ - cyanoäthyl) - carbonsäureamid als tiefgelbe Kristalle aus, die bei 159—160°C unter Zersetzung schmelzen.

Beispiel 4

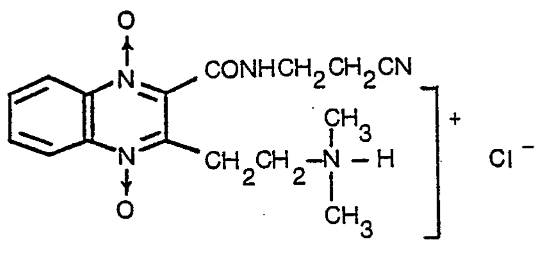

13,6 g (0,05 Mol) 1,4 - Dioxido - 3 - methyl - chinoxalin(2) - N - (2′ - cyanäthyl) - carbonsäureamid werden mit 10 g (0,1 Mol) 30%iger, wässriger Formaldehydlösung und 8,1 g (0,1 Mol) Dimethylaminhydrochlorid in 200 ml Acetonitril suspendiert. Anschliessend rührt mann 24 Stunden unter Rückfluss und filtriert dann 13,6 g (74,3%) der gelbweissen Kristalle von 1,4 - Dioxido - 3 - (2′ - dimethylaminoäthyl) - chinoxalin(2) - (2′ - cyanoäthyl) - carbonsäureamid - hydrochlorid heiss ab, die bei 211—213°C, unter Zersetzung schmelzen.

Beispiel 5

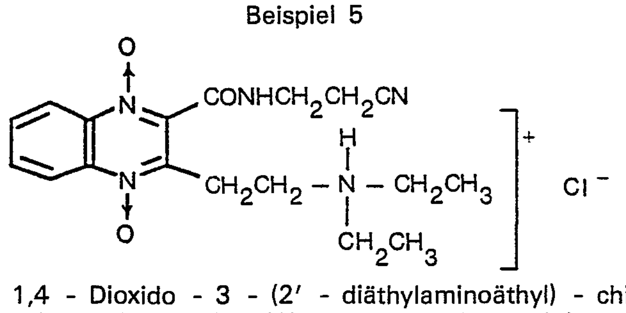

17,8 g (0,05 Mol) 1,4 - Dioxido - 3 - (2′ - diäthylaminoäthyl) - chinoxalin(2) - N - (2′ - cyanäthyl) - carbonsäureamid werden in 50 ml Wasser suspendiert und dann unter Kühlung bei 20°C in 50 ml 1 N Salzsäure gelöst. Die helle, klare Lösung wird zur Trockene eingedampft. Restfeuchtigkeit wird durch Trocknung über Phosphorpentoxid entzogen. Den zähviskosen Rückstand kristallisiert man aus Isopropanol/Aceton um und erhält 12,5 g (63,6%) 1,4-Dioxido-3-(2′-diäthylaminoäthyl) - chinoxalin(2) - n - (2′ - cyanoäthyl) - carbonsäureamid - hydrochloridals gelbweisse Kristalle, die bei 180—181°C schmelzen.

Beispiel 6

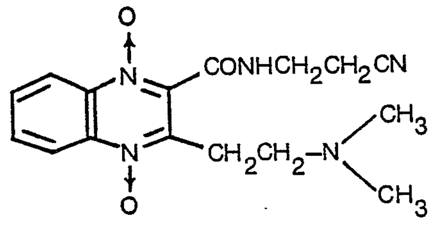

18,2 g (0,05 Mol) 1,4 - Dioxido - 3 - (2′ - dimethylaminoäthyl) - chinoxalin(2) - (2′ - cyanäthyl) - carbonsäureamid - hydrochlorid werden in 100 ml Wasser gelöst. Dann gibt man bei

einer Temperatur von 20°C 50 ml 1 N Natronlauge hinzu und extrahiert die Lösung mit Methylenchlorid. Aus der über Natriumsulfat getrockneten Methylenchloridphase erhält man dann 15,6 g (95%) 1,4 - Dioxido - 3 - (2' - dimethylaminoäthyl) - chinoxalin(2) - (2' - cyanäthyl) - carbonsäureamid, das bei 161°C unter Zersetzung schmilzt.

Folgende Verbindungen wurden analog den in den Beispielen beschriebenen Verfahren hergestellt bzw. sind herstellbar:

Verbindungstabelle 2

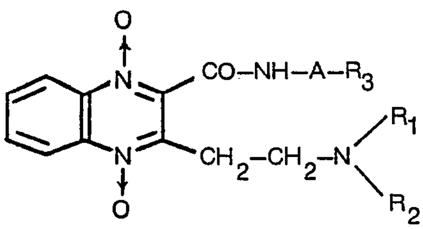

oder

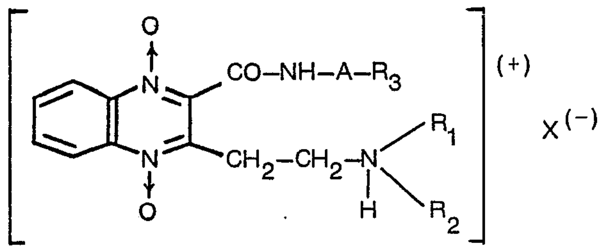

| Nr. | A | $R_1$ | $R_2$ | $R_3$ | X | Smp. in °C |
|---|---|---|---|---|---|---|
| 1 | $CH_2$ | $CH_3$ | $CH_3$ | CN | Cl | 179–181 |
| 2 | $CH_2$ | $CH_3CH_2$ | $CH_3CH_2$ | CN | – | 156–157 |
| 3 | $CH_2$ | $CH_3CH_2$ | $CH_3CH_2$ | CN | Cl | |
| 4 | $CH_2-CH_2$ | $CH_3(CH_2)_2$ | $CH_3(CH_2)_2$ | CN | – | 128–130 |
| 5 | $CH_2-CH_2$ | $CH_3(CH_2)_3$ | $CH_3(CH_2)_3$ | CN | – | 130–132 |
| 6 | $CH_2-CH_2$ | 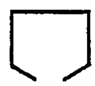 | | CN | – | 160–161 |
| 7 | $CH_2-CH_2$ | 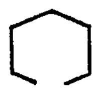 | | CN | – | 160–161 |
| 8 | $CH(CH_3)-CH_2$ | $CH_3$ | $CH_3$ | CN | Cl | |
| 9 | $CH(CH_3)-CH_2$ | $CH_3CH_2$ | $CH_3CH_2$ | CN | – | 136–138 |
| 10 | $CH(CH_3)-CH_2$ | $CH_3CH_2$ | $CH_3CH_2$ | CN | Cl | |
| 11 | $CH_2$ | $CH_3$ | $CH_3$ | CN | Br | 158–160 |
| 12 | $CH_2-CH_2$ | $CH_3$ | $CH_3$ | CN | – | 158–160 |
| 13 | $CH_2-CH_2$ | $CH_3$ | $CH_3$ | CN | Br | 202 (Zers.) |
| 14 | $CH_2-CH_2$ | 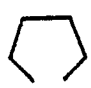 | | CN | Cl | 187–189 |
| 15 | $CH_2-CH_2$ | $CH_3$ | $CH_3$ | CN | Cl | 211–213 |
| 16 | $CH_2-CH_2$ | $CH_3$ | $CH_3$ | CN | Amber-list 15 | >300 |
| 17 | $CH_2-CH_2$ | $CH_3-CH_2$ | $CH_3-CH_2$ | CN | – | 158–159 |
| 18 | $CH_2-CH_2$ | $CH_3-CH_2$ | $CH_3-CH_2$ | CN | Cl | 180–181 |

| Nr. | A | $R_1$ | $R_2$ | $R_3$ | X | Smp. in °C |
|---|---|---|---|---|---|---|
| 19 | $CH_2-CH_2$ | $CH_3-CH_2$ | $CH_3-CH_2$ | CN | Amber-list 15 | >300 |
| 20 | $CH_2-CH_2$ | 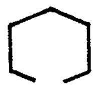 | | CN | Cl | 190–192 |
| 21 | $CH_2-CH_2$ | 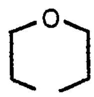 | | CN | Cl | 192 |
| 22 | $CH_2-CH_2$ | $CH_3$ | $CH_3$ | OH | Cl | 159–160 (Zers.) |
| 23 | $CH_2-CH_2$ | $CH_3$ | $CH_3$ | $S-CH_3$ | Cl | 185–186 |
| 24 | $CH_2-CH_2$ | $CH_3$ | $CH_3$ | $S-CH_3$ | – | 138–139 |
| 25 | $CH_2-CH_2$ | $CH_3$ | $CH_3$ | $OCH_3$ | Cl | 196–197 |
| 26 | $CH_2-CH_2$ | $CH_3$ | $CH_3$ | $OCH_3$ | – | 150–152 |
| 27 | $CH_2-CH_2$ | $CH_3$ | $CH_3$ | Br | Cl | 164–166 |
| 28 | $CH_2-CH_2$ | $CH_3$ | $CH_3$ | Cl | Cl | 194–196 (Zers.) |
| 29 | $CH_2-CH_2$ | $CH_3$ | $CH_3$ | Cl | – | 139–140 |
| 30 | $CH_2-CH_2$ | 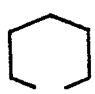 | | F | Cl | 182–183 (Zers.) |
| 31 | $CH_2-CH_2$ | 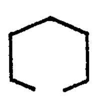 | | F | – | 174–175 |
| 32 | $CH_2-CH_2$ | $CH_3$ | $CH_3$ | H | Cl | 183–185 (Zers.) |
| 33 | $CH_2-CH_2$ | $CH_3$ | $CH_3$ | H | – | 153–155 |
| 34 | $CH_2-CH_2$ | 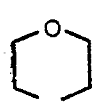 | | H | Cl | 188–190 (Zers.) |

| Nr. | A | $R_1$ | $R_2$ | $R_3$ | X | Smp. in °C |
|---|---|---|---|---|---|---|
| 35 | $CH_2-CH_2$ | 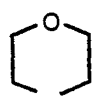 | | H | – | 185–186 |
| 36 | $CH_2-CH_2$ | $CH_3$ | $CH_3$ | F | Cl | 182 |
| 37 | $CH_2-CH_2$ | $CH_3$ | $CH_3$ | F | – | 166–167 |

Beispiel 7

Zur Herstellung von Endfutter enthaltend als Wirkstoff eine Verbindung der Formel I oder ein Säureadditionssalz davon (Formel Ia) in einer Konzentration von a) 25 ppm, b) 50 ppm, c) 200 ppm und d) 400 ppm werden folgende Futtervormischungen (Premix) zubereitet:

| | | | |
|---|---|---|---|
| a) | 0,15 | Gew.-Teile | einer der Verbindungen gemäss Formel I oder Ia |
| | 49,85 | Gew.-Teile | Bolus alba |
| | 150,0 | Gew.-Teile | Standardfutter für Geflügel, Schweine oder Wiederkäuer. |
| b) | 0,30 | Gew.-Teile | einer der Verbindungen gemäss Formel I oder Ia |
| | 44,70 | Gew.-Teile | Bolus alba |
| | 5,0 | Gew.-Teile | Kieselsäure |
| | 150,0 | Gew.-Teile | Standardfutter für Geflügel, Schweine oder Wiederkäuer. |
| c) | 1,2 | Gew.-Teile | einer der Verbindungen gemäss Formel I oder Ia |
| | 43,8 | Gew.-Teile | Bolus alba |
| | 5,0 | Gew.-Teile | Kieselsäure |
| | 150,0 | Gew.-Teile | Standardfutter für Geflügel, Schweine oder Wiederkäuer. |
| d) | 2,4 | Gew.-Teile | einer der Verbindungen gemäss Formel I oder Ia |
| | 47,6 | Gew.-Teile | Bolus alba |
| | 150,0 | Gew.-Teile | Standardfutter für Geflügel, Schweine oder Wiederkäuer. |

Die Wirkstoffe werden entweder direkt den Trägerstoffen zugemischt oder in einem geeigneten Lösungsmittel gelöst auf die Trägerstoffe aufgezogen. Anschliessend mahlt man zur gewünschten Teilchengrösse von z.B. 5—10 Mikron. Diese Futtervormischungen werden mit 5800 Gew.-Teilen Standardfutter vermischt oder zu 6000 Gew.-Teilen Fertigtränken verarbeitet.

Ausserdem können die Endfuttermischungen pelletiert werden (Futterpellets).

Die erfindungsgemässen Wirkstoffe der Formel I oder Ia werden dem Futter oder den Tränken für die Tiere in Mengen von 1 bis 500 ppm bezogen auf das Gesamtfutter bzw. -tränke entweder direkt oder in Form einer Vormischung zugesetzt.

Geeignete Vormischungen bestehen z.B. aus einer Mischung des Wirkstoffs beispielsweise mit Kaolin, Kalk, Aluminiumoxid, gemahlene Muschelschalen, Bolus alba, Aerosol, Stärke oder Nachmehle wie zum Beispiel Weizennachmehl. Zur Herstellung einer Futtermischung wird die nötige Menge Vormischung (Premix) der entsprechenden Menge eines handelsüblichen Standardfutters gründlich vermischt.

**Patentansprüche**

1. Chinoxalin-di-N-oxid-Derivate der allgemeinen Formel I

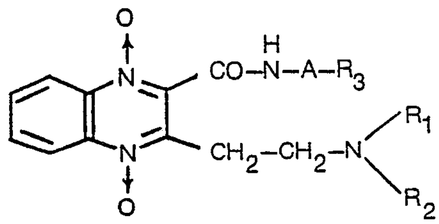

(I)

in welcher

$R_1$ und $R_2$ unabhängig voneinander geradkettiges oder verzweigtes Alkyl mit 1 bis 4 Kohlenstoffatomen oder zusammen mit dem N-Atom einen gegebenenfalls durch Alkyl mit 1 bis 4 Kohlenstoffatomen substituierten heterocyclischen Ring mit 4 bis 5 Ring-Kohlenstoffatomen und gegebenenfalls einem Sauerstoffatom als weiterem Heteroatom,

$R_3$ Wasserstoff, Methoxy, Methylthio, Hydroxy, Fluor, Chlor, Brom oder Cyan,

A Alkylen mit 1 bis 4 Kohlenstoffatomen bedeuten, mit der Massgabe dass wenn $R_3$ verschieden von Cyan ist, A 1,2-Aethylen bedeutet, einschliesslich ihrer Säureadditionssalze.

2. Chinoxalin-din-N-oxid-Derivate der allgemeinen Formel Ib

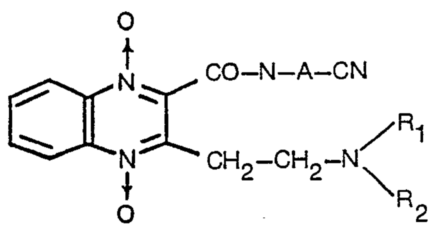

(Ib)

in welcher $R_1$ und $R_2$ unabhänging voneinander $C_1$—$C_4$-Alkyl oder zusammen mit dem N-Atom einen gegebenenfalls durch $C_1$—$C_4$-Alkyl substituierten heterocyclischen Ring mit 4 bis 5 Ring-Kohlenstoffatomen und gegebenenfalls einem Sauerstoffatom als weiterem Heteroatom darstellen, und A Alkylen mit 1 bis 4 Kohlenstoffatomen bedeutet, einschliesslich ihrer Säureadditionssalze.

3. Chinoxalin-di-N-oxidsalz der Formel

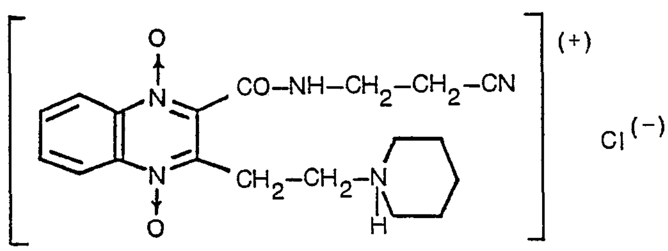

4. Chinoxalin-di-N-oxidsalz der Formel

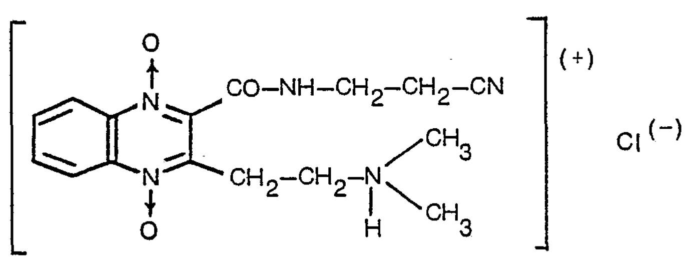

5. Chinoxalin-di-N-oxidsalz der Formel

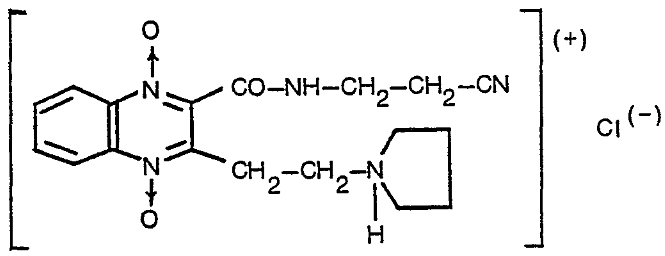

6. Chinoxalin-di-N-oxidsalz der Formel

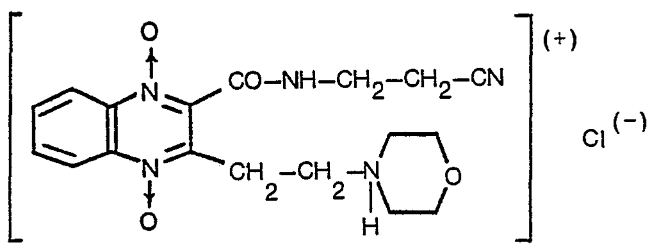

7. Chinoxalin-di-N-oxid der Formel

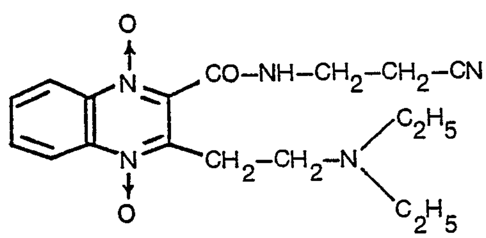

VII

8. Chinoxalin-di-N-oxid Salz der Formel VII

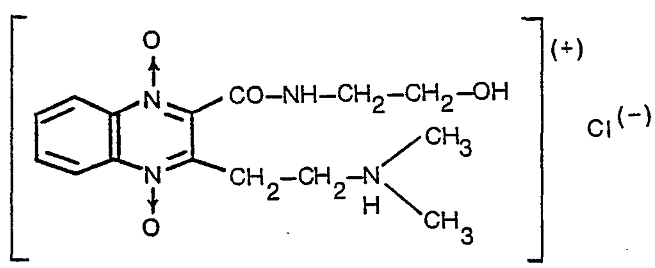

(VII)

9. Verfahren zur Herstellung einer Verbindung der Formel I oder eines ihrer Säureadditionssalze gemäss Anspruch 1, dadurch gekennzeichnet, dass man eine Verbindung der Formel II

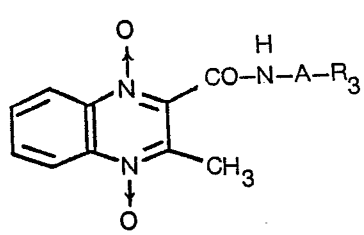

(II)

in welcher $R_3$ und A die in Anspruch 1 angegebenen Bedeutungen besitzen mit einem sekundären Amin der Formel III.

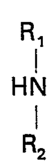

(III)

oder einem seiner Säureadditionssalze der Formel IIIa

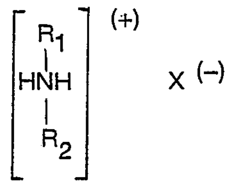

in welchen $R_1$ und $R_2$ die in Anspruch 1 angegebenen Bedeutungen besitzen und $X^{(-)}$ das Anion einer geeigneten anorganischen oder organischen Säure darstellt, zusammen mit Formaldehyd oder einem Formaldehyd abspaltenden Mittel in Gegenwart von inerten Lösungsmitteln umsetzt, wobei gegebenenfalls das erhaltene Endprodukt, falls es eine Verbindung der Formel I darstellt, durch Umsetzung mit einer geeigneten Säure in eine Verbindung der Formel Ia

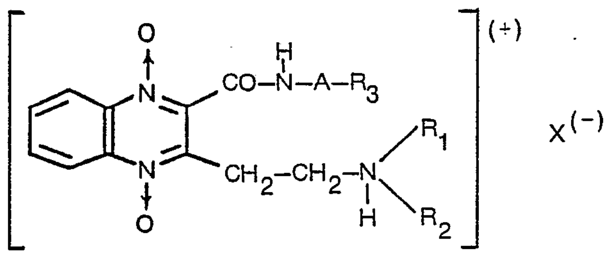

(Ia),

in welcher $R_1$, $R_2$, $R_3$ und A die unter Formel I in Anspruch 1 angegebenen Bedeutungen besitzen und $X^{(-)}$ das Anion einer geeigneten anorganischen oder organischen Säure darstellt, oder falls es eine Verbindung der Formel Ia darstellt, durch Umsetzung mit einer geeigneten Base in eine Verbindung der Formel I überführt wird.

10. Verfahren nach Anspruch 9 dadurch gekennzeichnet, dass man für den Fall, dass $R_3 = Br$ und $A = -CH_2-CH_2-$ sind, als Ausgangssubstanz eine Verbindung der Formel II'

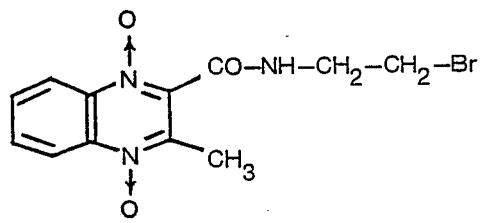

(II')

einsetzt, welche durch Umsetzung eine Verbindung der Formel IV

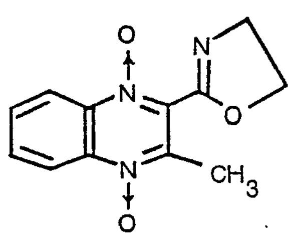

(IV)

mit wässriger 48%iger Bromwasserstoffsäure erhalten wurde.

11. Verfahren nach Anspruch 9 dadurch gekennzeichnet, dass man für den Fall, dass $R_3 = Cl$ und $A = CH_2-CH_2-$ ist, als Ausgangssubstanz eine Verbindung der Formel II''

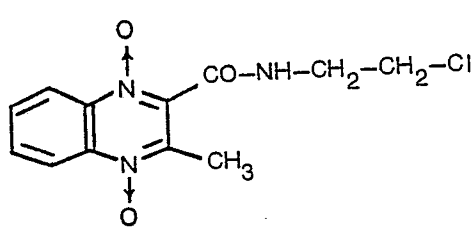

(II'')

einsetzt, welche durch Umsetzung einer Verbindung der Formel IV

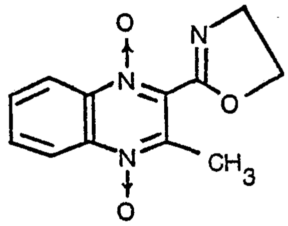

(IV)

mit wässriger konzentrierter Salzsäure erhalten wurde.

12. Mittle zur Bekämpfung schädlicher Mikroorganismen enthaltend als Wirkstoff mindestens eine Verbindung der Formel I oder eines ihrer Säureadditionssalze gemäss Anspruch 1 zusammen mit inerten Trägerstoffen und/oder Verteilungsmitteln.

13. Mittel zur Förderung des Wachstums von Haus- und Nutztieren enthaltend als Wirkstoff mindestens eine Verbindung der Formel I oder eines ihrer Säureadditionssalze gemäss Anspruch 1 zusammen mit inerten Trägerstoffen und/oder Verteilungsmitteln.

## Claims

1. A quinoxaline-di-N-oxide derivative of the general formula I

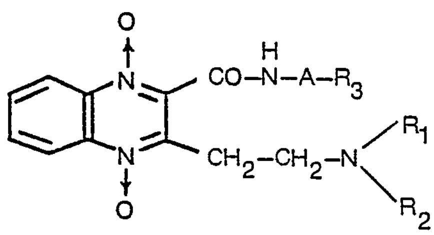

(I)

in which

$R_1$ and $R_2$ independently of one another are each straight-chain or branched-chain alkyl having 1 to 4 carbon atoms, or together with the N atom form a heterocyclic ring which is unsubstituted or substituted by alkyl having 1 to 4 carbon atoms, and which has 4 to 5 ring carbon atoms and optionally an oxygen atom as further hetero atom,

$R_3$ is hydrogen, methoxy, methylthio, hydroxyl, fluorine, chlorine, bromine or cyano, and

A is alkylene having 1 to 4 carbon atoms, with the proviso that if $R_3$ has a meaning other than cyano, A is 1,2-ethylene,

including the acid addition salts thereof.

2. A quinoxaline-di-N-oxide derivative of the general formula Ib

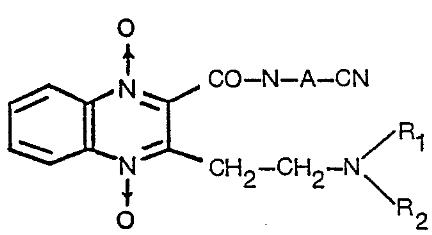

(Ib)

in which $R_1$ and $R_2$ independently of one another are each $C_1$—$C_4$-alkyl, or together with the N atom form a heterocyclic ring which is unsubstituted or substituted by $C_1$—$C_4$-alkyl and which has 4 to 5 ring carbon atoms and optionally an oxygen atom as further hetero atom, and A is alkylene having 1 to 4 carbon atoms, including the acid addition salts thereof.

3. A quinoxaline-di-N-oxide salt of the formula

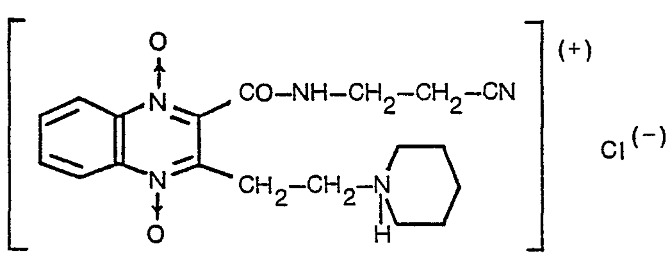

4. A quinoxaline-di-N-oxide salt of the formula

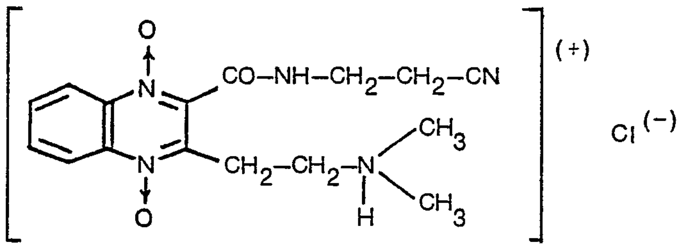

5. A quinoxaline-di-N-oxide salt of the formula

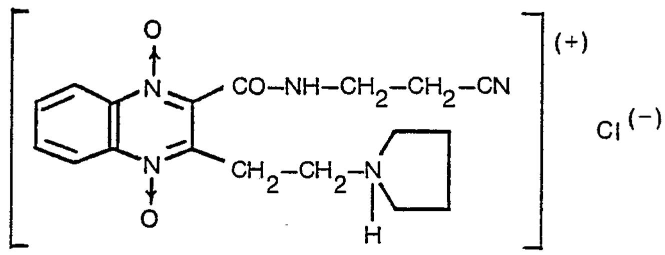

6. A quinoxaline-di-N-oxide salt of the formula

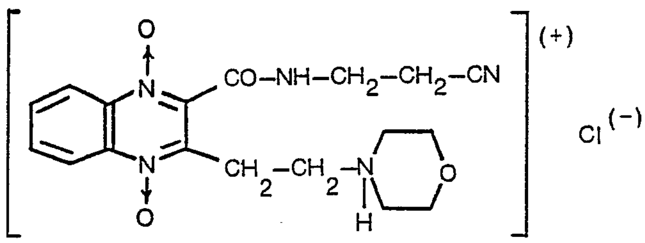

7. A quinoxaline-di-N-oxide of the formula

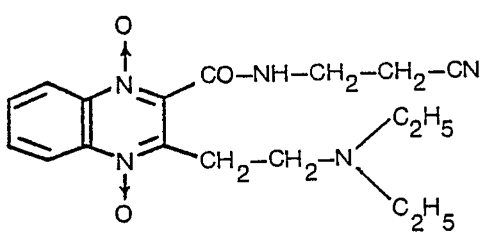

8. A quinoxaline-di-N-oxide salt of the formula VII

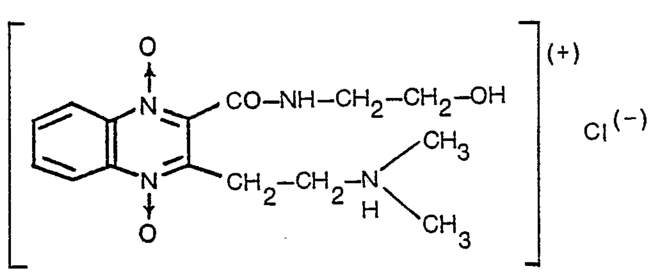

(VII)

9. A process for producing a compound of the formula I or one of the acid addition salts thereof, according to Claim 1, which process comprises reacting a compound of the formula II

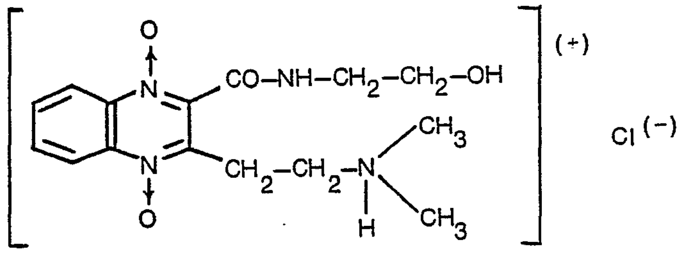

(VII)

in which $R_3$ and A have the meanings defined in Claim 1, with a secondary amine of the formula III

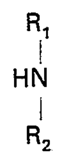

(III),

or with one of its acid addition salts of the formula IIIa

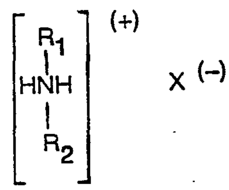

(IIIa)

in which $R_1$ and $R_2$ have the meanings defined in Claim 1, and $X^{(-)}$ is the anion of a suitable inorganic or organic acid, together with formaldehyde or with an agent splitting off formaldehyde, in the presence of inert solvents, whereby optionally in the final product obtained, if it is a compound of the formula I, is converted, by reaction with a suitable acid, into a compound of the formula Ia

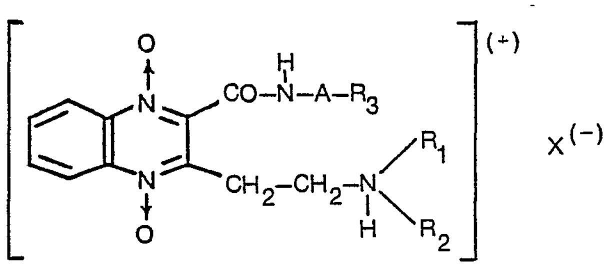

(Ia),

in which $R_1$, $R_2$, $R_3$ and A have the meanings defined under the formula I, and $X^{(-)}$ is the anion of a suitable inorganic or organic acid, or, if it is a compound of the formula Ia, is converted, by reaction with a suitable base, into a compound of the formula I.

10. A process according to Claim 9, wherein there is used as starting material, in the case where $R_3 = Br$ and $A = -CH_2-CH_2-$, a compound of the formula II′

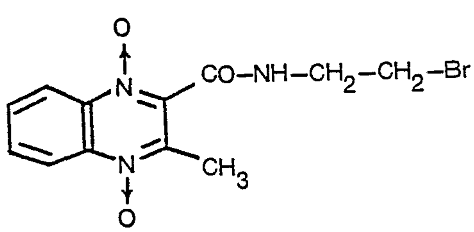

(II′)

which has been obtained by reaction of a compound of the formula IV

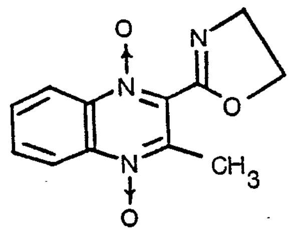

(IV)

with 48% aqueous hydrobromic acid.

11. A process according to Claim 9, wherein there is used as starting material, in the case where $R_3 = Cl$ and $A = CH_2—CH_2—$, a compound of the formula II''

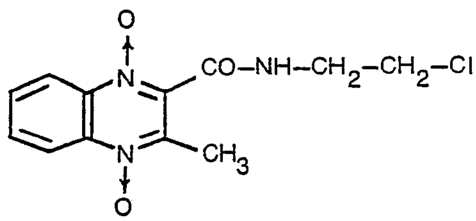

(II'')

which has been obtained by reaction of a compound of the formula IV

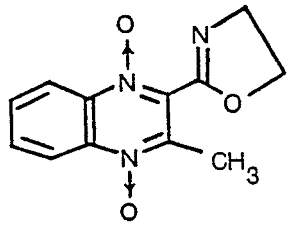

(IV)

with aqueous concentrated hydrochloric acid.

12. A composition for controlling harmful microorganisms, which contains as active ingredient at least one compound of the formula I, or one of its addition salts, according to Claim 1, together with inert carriers and/or distributing agents.

13. A composition for promoting the growth of domestic animals and productive animals, which composition contains as active ingredient at least one compound of the formula I, or one of its acid addition salts, according to Claim 1, together with inert carriers and/or distributing agents.

**Revendications**

1. Dérivés de di-N-oxydes de quinoxalines de formule générale (I):

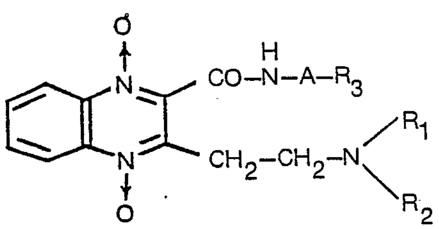

(I)

dans laquelle:

$R_1$ et $R_2$ représentent chacun, indépendamment l'un de l'autre, un groupe alkyle à chaîne droite ou ramifiée en $C_1—C_4$ ou forment ensemble et avec l'atome d'azote un noyau hétérocyclique éventuellement substitué par un groupe alkyle en $C_1—C_4$ et contenant 4 à 5 atomes de carbone cycliques et le cas échéant un atome d'oxygène comme autre hétéroatome,

$R_3$ représente l'hydrogène, un groupe méthoxy, méthylthio, hydroxy, le fluor, le chlore, le brome ou un groupe cyano,

A représente un groupe alkylène contenant de 1 à 4 atomes de carbone, étant spécifié que lorsque $R_3$ a une signification autre que le groupe cyano, A représente le groupe 1,2-éthylène,

y compris leurs sels formés par addition avec des acides.

2. Dérivés de di-N-oxydes de quinoxalines de formule générale (Ib):

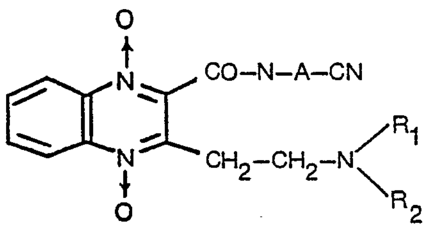

(Ib)

dans laquelle:

$R_1$ et $R_2$ représentent chacun, indépendamment l'un de l'autre, un groupe alkyle en $C_1$—$C_4$ ou forment ensemble et avec l'atome d'azote un noyau hétérocyclique éventuellement substitué par un groupe alkyle en $C_1$—$C_4$ et contenant 4 à 5 atomes de carbone cycliques et le cas échéant un atome d'oxygène comme autre hétéroatome, et

A représente un groupe alkylène contenant de 1 à 4 atomes de carbone,

y compris leurs sels formés par addition avec des acides.

3. Le sel de di-N-oxyde de quinoxaline de formule:

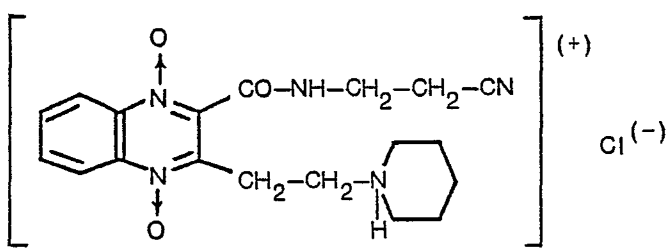

4. Le sel de di-N-oxyde de quinoxaline de formule:

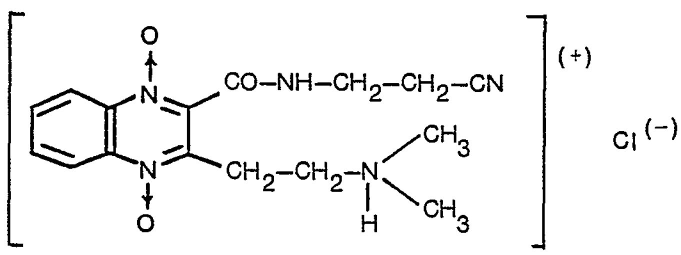

5. Le sel de di-N-oxyde de quinoxaline de formule:

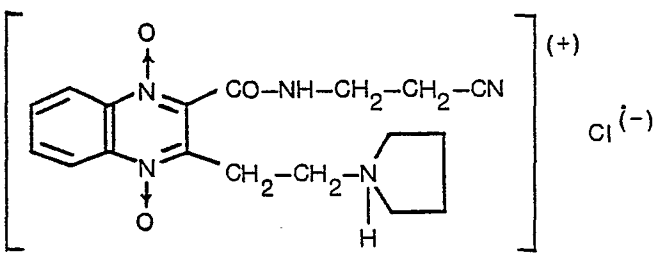

6. Le sel de di-N-oxyde de quinoxaline de formule:

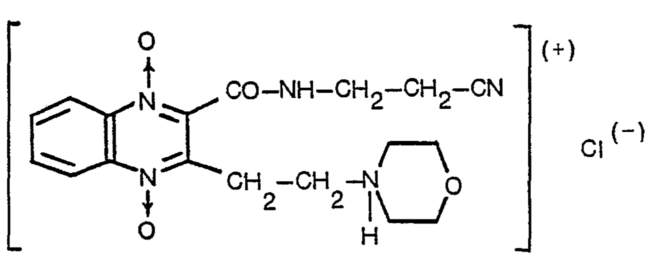

7. Le di-N-oxyde de quinoxaline de formule:

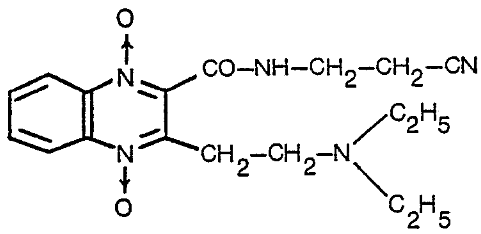

8. Le sel de di-N-oxyde de quinoxaline de formule (VII):

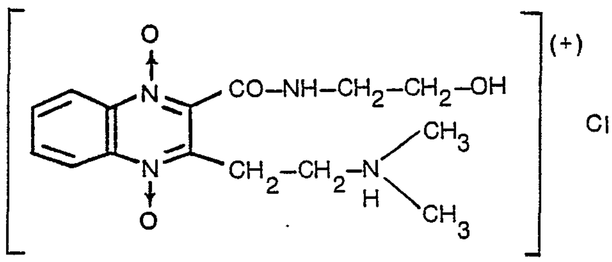 $Cl^{(-)}$ (VII)

9. Procédé de préparation d'un composé de formule (I) ou d'un de ses sels formés par addition avec un acide selon la revendication 1, caractérisé en ce que l'on fait réagir un composé de formule (II):

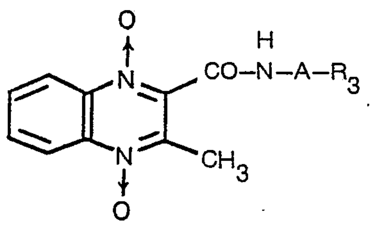 (II)

dans laquelle $R_3$ et A ont les significations indiquées dans la revendication 1, avec une amine secondaire de formule (III):

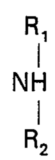 (III)

ou l'un de ses sels formés par addition avec un acide, de formule (IIIa):

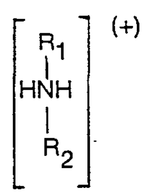 $X^{(-)}$ (IIIa)

dans lesquels:

$R_1$ et $R_2$ ont les significations indiquées dans la revendication 1 et

$X^{(-)}$ représente l'anion d'un acide organique ou minéral approprié, avec le formaldéhyde ou un agent libérant du formaldéhyde en présence de solvants inertes, et le cas échéant, on convertit le produit final obtenu, lorsqu'il s'agit d'un composé de formule (I), par réaction avec un acide approprié, en un composé de formule (Ia)

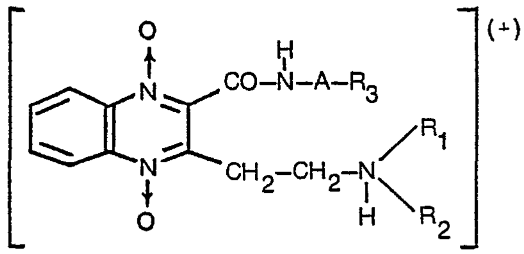

$X^{(-)}$ (Ia),

dans laquelle

$R_1$, $R_2$, $R_3$ et A ont les significations indiquées en référence à la formule (I) de la revendication 1, et $X^{(-)}$ représente l'anion d'un acide minéral ou organique approprié, ou bien, orsqu'il s'agit d'un composé de formule (Ia), on le convertit en un composé de formule (I) par réaction avec une base appropriée.

10. Procédé selon la revendication 9, caractérisé en ce que, dans le cas où $R_3 = Br$ et $A = —CH_2—CH_2—$, on utilise en tant que produit de départ un composé de formule (II')

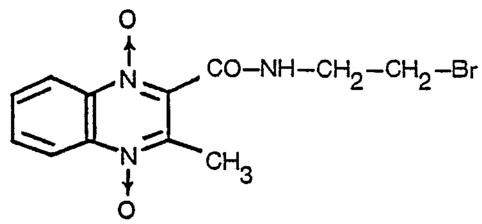

(II')

qui a été obtenu en faisant réagir un composé de formule (IV):

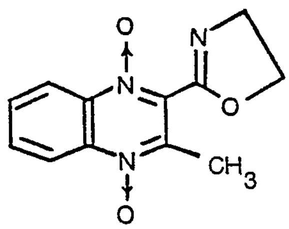

(IV)

avec l'acide bromhydrique aqueux à 48%.

11. Procédé selon la revendication 9, caractérisé en ce que, dans le cas où $R_3 = Cl$ et $A = CH_2—CH_2—$, on utilise comme produit de départ un composé de formule (II''):

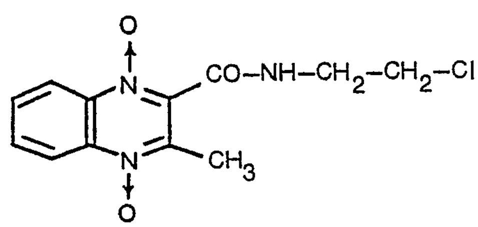

(II'')

qui a été obtenu en faisant réagir un composé de formule (IV):

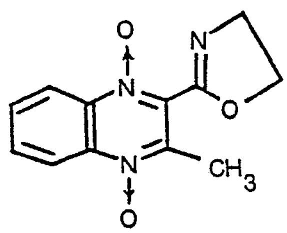

(IV)

avec l'acide chlorhydrique aqueux concentré.

12. Produit servant à combattre les microorganismes nuisibles, contenant en tant que substance active au moins un composé de formule (I) ou l'un de ses sels formés par addition avec un acide, selon la revendication 1, avec des véhicules et/ou diluants inertes.

13. Produit pour activer la croissance des animaux familiers et utiles, contenant en tant que substance active, au moins un composé de formule (I) ou l'un de ses sels formé par addition avec un acide selon la revendication 1, avec des véhicules et/ou diluants inertes.